(19) Europäisches Patentamt
European Patent Office
Office européen des brevets

(11) Publication number: 0 056 314 B1

(12) **EUROPEAN PATENT SPECIFICATION**

(45) Date of publication of patent specification: **21.08.85**

(51) Int. Cl.[4]: **C 07 C 145/02,** A 01 N 37/28, A 01 N 37/34

(21) Application number: **82300094.8**

(22) Date of filing: **08.01.82**

(54) **N-Haloalkyl thiobenzanilides, production thereof, compositions containing them and their use as fungicides.**

(30) Priority: **08.01.81 US 223469**
**08.01.81 US 223470**

(43) Date of publication of application:
**21.07.82 Bulletin 82/29**

(45) Publication of the grant of the patent:
**21.08.85 Bulletin 85/34**

(84) Designated Contracting States:
**AT BE CH DE FR GB IT LI NL SE**

(56) References cited:
**EP-A-0 001 480**
**US-A-4 097 512**

(73) Proprietor: **STAUFFER CHEMICAL COMPANY**
**Westport Connecticut 06880 (US)**

(72) Inventor: **Chin, Hsiao-Ling Mao**
**2736 San Antonio Drive**
**Walnut Creek California 94598 (US)**
Inventor: **Pallos, Ferenc Marcus**
**136 Twin Peaks Avenue**
**Walnut Creek California 94595 (US)**

(74) Representative: **Smith, Sydney et al**
**Elkington and Fife High Holborn House**
**52/54 High Holborn**
**London WC1V 6SH (GB)**

EP 0 056 314 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to certain novel N-haloalkyl thiobenzanilides, to the production thereof, to compositions containing them and to their use as fungicides.

According to EP—A— 1480, N— 1,1,2,2-tetrachloro-2-fluoroethyl-thio-benzanilide is active as a fungicide and an acaricide.

Also, U.S.—A 4,097,512 discloses the use of certain N-tetrachlorofluoroethylthio-halo-benzoyl-anilide compounds as fungicides.

The present invention provides as new compounds having fungicidal activity, compounds of the general formula:—

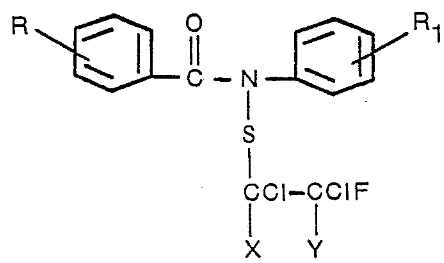

in which R is hydrogen, $C_1$—$C_5$ alkyl, preferably $C_1$—$C_3$ alkyl, $C_1$—$C_3$ haloalkyl, preferably —$CF_3$, halogen including —F, —Cl, —Br, and —I, —$NO_2$, $C_1$ to $C_2$ alkoxy, $R_1$ is —H or —C≡N, and X and Y are either —Cl or —F but not identical when $R_1$ is —H and, X and Y are —Cl when $R_1$ is —C≡N.

Unexpectedly, the present compounds are advantageous in that they are generally effective at lower concentrations, particularly in relation to bean rust, bean mildew, tomato blight and blue grass leaf spot, for example.

Although the compounds of the invention are generally active as fungicides, it has been discovered that they are also effective as systemic toxicants and this ancillary feature greatly increases their usefulness and versatility in treating fungus-infected food crops. As those skilled in the art are aware, a systemic biocide is taken up internally by the organism to which it is applied and lodges in the tissues while still retaining toxicological properties. When used to protect food crops systemic toxicants are not subject to weathering since they are confined whithin the interstices of the plant tissues which are thereby internally immunized against the attack of harmful fungi, blights and similar pesticidal microorganisms.

The compounds of the present invention are prepared by the following general reaction.

Reaction No. 1

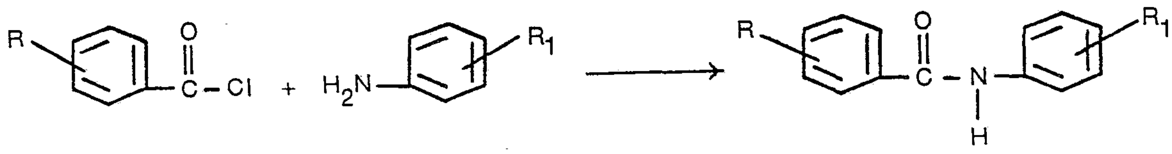

Generally a molar amount of the substituted benzoyl chloride reactant, dissolved in benzene, is added to a mixture of the aniline reactant and a slight molar excess of an HCl acceptor such as triethylamine. The mixture is refluxed for 1/2 hour and then cooled. The solid reaction product is diluted with a solvent such as ethyl acetate or chloroform and washed with water twice and salt solution once. The final product is dried over $MgSO_4$, filtered and evaporated.

Reaction No. 2

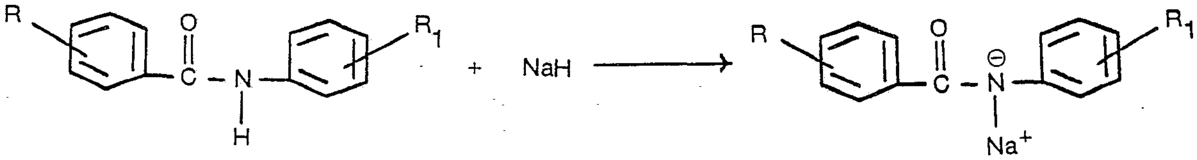

Under a dry nitrogen atmosphere a molar amount of the reaction product from Reaction No. 1 is dissolved in dry tetrahydrofuran (THF). Next, a slight molar excess of NaH is added with stirring. The mixture is refluxed for 1 hour and cooled.

Reaction No. 3

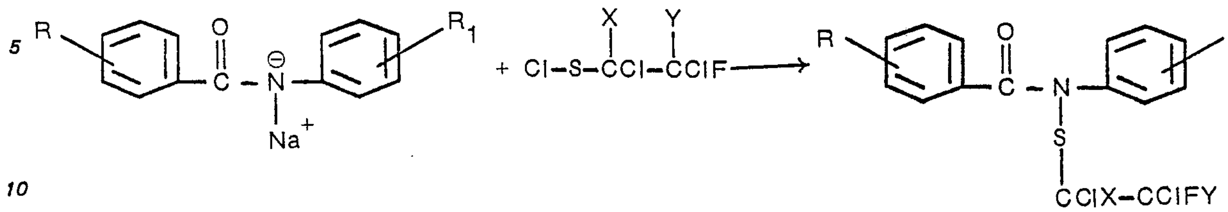

A molar amount of

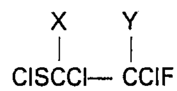

in THF is added dropwise to the reaction mixture of Reaction No. 2. The mixture is refluxed for 2 1/2 hours and a large amount of $CH_2Cl_2$ is added to the solid reaction product. The product is washed twice with water, dried over $MgSO_4$ and evaporated.

The

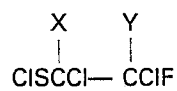

is prepared as outlined below:

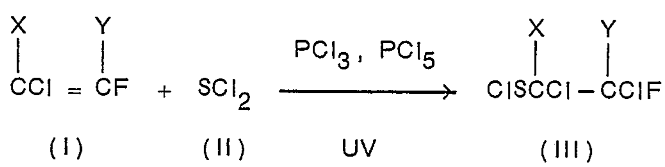


An equimolar mixture of Reagent I and Reagent II is irradiated under a U V lamp (at 8°C to 60°C) for 3 to 5 hours. The product III is isolated and purified by distillation under reduced pressure (40 mm Hg to 50 mm Hg).

Preparation of compounds of this invention is illustrated by the following examples.

Example I

Preparation of 2-iodobenzoyl anilide

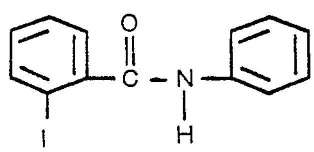

43.2 g. (0.464 moles) aniline and 46.9 g. triethylamine are mixed in 930 ml. benzene by stirring. A solution of 123.7 g (0.464 moles) 2-iodobenzoyl chloride in 450 ml. benzene is added to the first mixture through a dropping funnel. An exothermic reaction takes place. After the addition is finished, the mixture is refluxed for 1/2 hour. The mixture is cooled and the product solidifies. The product is filtered, added to water, and stirred to dissolve triethylamine hydrochloride. The product is filtered again and dried to yield 146.8 g of the desired product. (98% yield) m.p. 141—144°C.

Example II

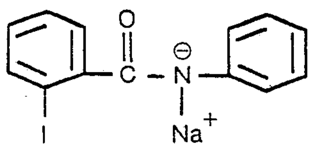

8.075 g. (0.025 moles) of the amide reaction product of Example I is dissolved in 60 ml. of dry THF under a dry nitrogen atmosphere. 0.66 g. (0.0275 moles) NaH is added to the mixture with stirring. The mixture is refluxed for one hour and then cooled.

Example III

N—1,1,2-trichloro-2,2-difluoromethylthio-o-iodobenzanilide

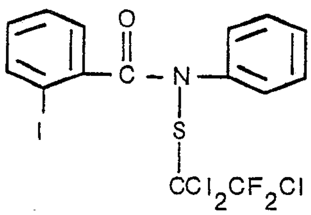

(0.025 moles) of $ClSCCl_2CF_2Cl$ dissolved in 12 ml. THF is added dropwise to the cooled reaction mixture of Example II. The mixture is then refluxed for 2—1/2 hours. Next 150 ml. $CH_2Cl_2$ is added, the mixture is washed twice with water, dried over $MgSO_4$ and evaporated to yield the desired product.

Example IV

Preparation of 2-iodobenzoyl anilide

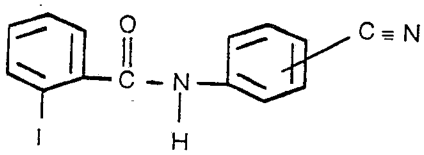

43.2 g. (0.464 moles) aniline and 46.9 g. triethylamine are mixed in 930 ml. benzene by stirring. A solution of 123.7 g. (0.464 moles) 2-iodobenzoyl chloride in 450 ml. benzene is added to the first mixture through a dropping funnel. An exothermic reaction takes place. After the addition is finished, the mixture is refluxed for 1/2 hour. The mixture is cooled and the product solidifies. The product is filtered, added to water, and stirred to dissolve triethylamine hydrochloride. The product is filtered again and dried to yield 146.8 g. of the desired product. (98% yield) m.p. 141—144°C.

Example V

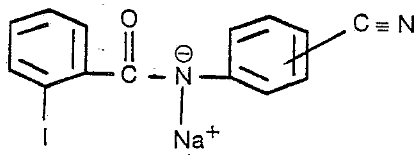

8.075 g. (0.025 moles) of the amide reaction product of Example I is dissolved in 60 ml. of dry THF under a dry nitrogen atmosphere. 0.66 g. (0.0275 moles) NaH is added to the mixture with stirring. The mixture is refluxed for one hour and then cooled.

Example VI

N-2-fluorotetrachloroethylthio-o-iodo-p'-cyanobenzanilide

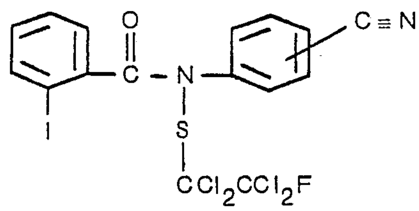

(0.025 moles) of $ClSCCl_2CCl_2F$ dissolved in 12 ml. THF is added dropwise to the cooled reaction mixture of Example II. The mixture is then refluxed for 2—1/2 hours. Next 150 ml. $CH_2Cl_2$ is added, the mixture is washed twice with water, dried over $MgSO_4$ and evaporated to yield the desired product.

The following is a table of certain selected compounds that are prepared according to the procedure described herein. Compound numbers as assigned to each compound and are used throughout the remainder of the application.

TABLE I

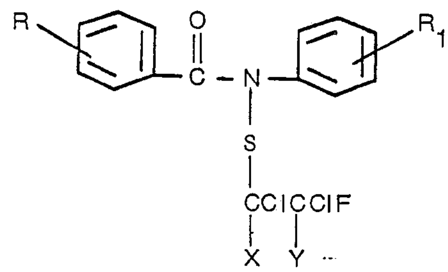

| Compound Number | R | $R_1$ | X | Y | Physical Constants m.p. or $nD^{30}$ |
|---|---|---|---|---|---|
| 1 | 2—I | H | Cl | F | glassy solid |
| 2 | H | H | Cl | F | semi-solid |
| 3 | H | H | F | Cl | 1.5772 |
| 4 | 2—$CF_3$ | H | F | Cl | 1.5345 |
| 5 | 2—$CF_3$ | H | Cl | F | 1.5410 |
| 6 | 2—I | H | F | Cl | 1.6034 |
| 7 | 2—$OCH_3$ | H | F | Cl | 1.5698 |
| 8 | 2—I | C≡N | Cl | Cl | 98—100.5°C |

Foliar Fungicide Evaluation Tests

A. *Evaluation for Preventive Action*

1. *Bean Rust Test:* Pinto bean plants (*Phaseolus vulgaris* L.) approximately 10 centimeters (cm.) tall are transplanted into sandy loam soil in three-inch clay pots. The plants are then inverted and dipped for two to three seconds in 50—50 acetone/water solution of the test chemical. Test concentrations range from 1000 ppm downward. After the leaves are dried, they are inoculated with a water suspension of spores of the bean rust fungus (*Urumyces phaseoli* Arthur) and the plants are placed in an environment of 100% humidity for 24 hours. The plants are then removed from the humidity chamber and held until disease pustules appear on the leaves. Effectiveness is recorded as the lowest concentration, in ppm, which will provide 75% or greater reduction in pustule formation as compared to untreated, inoculated plants. These values are recorded in Table II.

2. *Bean Powdery Mildew Test:* A candidate chemical is prepared and applied in the same manner as for the bean rust test. After the plants are dry, the leaves are dusted with spores of the powder mildew fungus(*Erysiphe polygoni* De Candolle) and the plants are retained in the greenhouse until the fungal growth appears on the leaf surface. Effectiveness is recorded as the lowest concentration, in ppm, which will provide 75% or greater reduction in mycelial formation as compared to untreated, inoculated plants. These values are recorded in Table II.

3. *Tomato Early Blight*: A candidate compound is dissolved in an appropriate solvent and diluted with a 50—50 acetone water solution. Four week old tomato (*Lycopersicon esculentum*) plants are then sprayed with the solution to the point of runoff. Test concentrations range from 1000 ppm downward. When the leaves are dry, they are innoculated with a water suspension of spores of the early blight fungus (*Alternaria solani* Ellis and Martin) and placed in an environment of 100% humidity for 48 hours. The plants are then removed from the humidity chamber and held until disease lesions appear on the leaves. Effectiveness is recorded as the lowest concentration, in ppm, which will provide 75% or greater reduction in the number of lesions formed as compared to untreated, inoculated plants. These values are recorded in Table II.

4. *Blue Grass Leaf Spot:* A candidate chemical is prepared and applied in the same manner as the tomato early blight test except that four week old Kentucky Bluegrass (*Poa pratensis*) plants are utilized as the host plant. When the leaves are dry, they are inoculated with water suspension of spores of the blue grass leaf spot fungus (*Helminthosporium sativum*) and placed in an environment of 100% humidity for 48 hours. The plants are then removed from the humidity chamber and held until disease lesions appear on the leaves. Effectiveness is recorded as the lowest concentration, in ppm, which will provide 75% or greater reduction in number of lesions formed as compared to untreated, inoculated plants. These values are recorded in Table II.

B. *Evaluation for Eradicant Action*

1. *Bean Rust Test:* Untreated bean plants (*Phaseolus vulgaris* L.) are inoculated with spores of the bean rust fungus (*Uromyces phaseoli* Arthur) and placed in an environment with 100% humidity for 24 hours. The plants are then removed from the humidity chamber and held in the greenhouse for two days to allow the disease to become established. A candidate chemical is then prepared and applied in the same manner as in the bean rust test in "Evaluation for Preventive Action". Eradicative effectiveness is recorded as the lowest concentration, in ppm, which will provide a 75% or greater reduction in number of pustules appearing on the leaves as compared to untreated inoculated plants. These values are recorded in Table II.

2. *Bean Powdery Mildew Test*: Untreated pinto bean plants are dusted with spores of the powdery mildew fungus (*Erysiphe polygoni* De Candolle) and maintained in the greenhouse until mycelial growth appears in the leaf surface. A candidate chemical is then prepared and applied in the same manner as for the bean rust test. Four days later the leaves are examined for inhibition of further mycelial growth. Eradicative effectiveness is recorded as the lowest concentration, in ppm, which will provide a 75% or greater inhibition of viable, sporulating mycelium as compared to untreated inoculated plants. These values are recorded in Table II.

TABLE II

Preventive Action

| Compound Number | Bean Rust | Bean Powdery Mildew | Tomato Early Blight | Blue Grass Leaf Spot |
|---|---|---|---|---|
| 1 | 10 | 100 | 100 | 25 |
| 2 | 25 | 50 | 50 | 25 |
| 3 | 50 | 50 | 25 | 25 |
| 4 | 25 | 100 | 50 | 50 |
| 5 | 10 | 100 | 50 | 100 |
| 6 | 10 | 100 | 50 | * |
| 7 | 50 | 50 | 50 | 100 |
| 8 | 100 | >1000 | 20 | 20 |

* No control at 1000 ppm and not tested at higher concentrations.

TABLE II (continued)

Eradicant Action

| Compound Number | Bean Rust | Bean Powdery Mildew |
|---|---|---|
| 1 | 1 | >50 |
| 2 | >50 | >50 |
| 3 | >50 | >50 |
| 4 | 0.5 | 50 |
| 5 | 0.5 | (50) |
| 6 | 0.5 | >50 |
| 7 | — | — |

( ) Indicates partial control.

The compounds of this invention are generally embodied into a form suitable for convenient application. For example, the compound can be embodied into a pesticidal composition which is provided in the form of emulsions, suspensions, solutions, dusts and aerosol sprays. In general, such compositions will contain, in addition to the active compound, the adjuvants which are found normally in pesticide preparations. In these compositions, the active compound of this invention can be employed as the sole pesticide component or it can be used in admixture with other compounds having similar uility. The pesticide compositions of this invention can contain, as adjuvants, organic solvents, such as sesame oil, xylene range solvents, heavy petroleum, etc.; water; emulsifying agents; surface active agents, talc; pyrophyllite; diatomite; gypsum; clays, propellants, such as dichlorodifluoromethane, etc. If desired, however, the active compound can be applied directly to feedstuffs, seeds, etc., upon which the pests feed. When applied in such a manner, it will be advantageous to use a compound which is not volatile. In connection with the activity of the presently disclosed pesticidal compound, it should be fully understood that is is not necessary that they be active as such. The purposes of this invention will be fully served if the compound is rendered active by external influences, such as light or by some physiological action which occurs when the compound is ingested into the body of the pest.

The precise manner in which the pesticidal compositions of this invention are used in any particular instance will be readily apparent to a person skilled in the art. Generally, the active pesticide compound will be embodied in the form of a liquid composition; for example, an elusion, suspension, or aerosol spray. While the concentration of the active pesticide in the present compositions can vary within rather wide limits, ordinarily the pesticide compound will comprise not more than about 15.0% by weight of the composition. Preferably, however, the pesticide compositions of this invention will be in the form of solutions or suspensions containing about 0.1 to 1.0% by weight of the active pesticide compound.

**Claims for the Contracting States: BE CH DE FR GB IT LI NL SE**

1. Compounds of the general formula:—

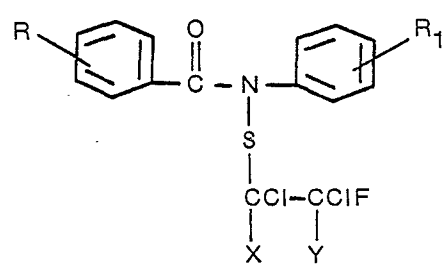

in which R is hydrogen, $C_1$—$C_5$ alkyl, $C_1$—$C_3$ haloalkyl, halogen, —$NO_2$, $C_1$—$C_2$ alkoxy, $R_1$ is —H or —C≡N, and X and Y are either —Cl or —F provided they are not identical when $R_1$ is —H and, X and Y are —Cl when $R_1$ is —C≡N.

2. A compound as claimed in claim 1 characterised in that R is 2—I, $R_1$ is —H, X is —Cl and Y is —F.

3. A compound as claimed in claim 1 characterised in that R is —H, $R_1$ is —H, X is —Cl and Y is —F.

4. A compound as claimed in claim 1 characterised in that R is —H, $R_1$ is —H, X is —F and Y is —Cl.

5. A compound as claimed in claim 1 characterised in that R is 2—$CF_3$, $R_1$ is —H, X is —F and Y is —Cl.
6. A compound as claimed in claim 1 characterised in that R is 2—$CF_3$, $R_1$ is —H, X is —Cl and Y is —F.
7. A compound as claimed in claim 1 characterised in that R is 2—I, $R_1$ is —H, X is —F and Y is —Cl.
8. A compound as claimed in claim 1 characterised in that R is 2—$OCH_3$, $R_1$ is —H, X is —F and Y is —Cl.
9. A compound as claimed in claim 1 characterised in that R is 2—I, $R_1$ is —C≡N, X is —Cl, and Y is —Cl.
10. A method of making a compound as claimed in claim 1 wherein a compound of the general formula

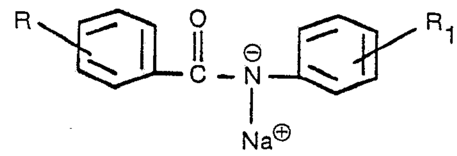

in which R and $R_1$ have the meaning given in claim 1, is reacted with a compound of the general formula

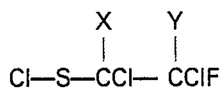

where X and Y have the meaning given in claim 1.

11. A fungicidal composition comprising a fungicidally effective amount of a compound as claimed in any of claims 1 to 9 or when made by the method of claim 10, in association with an inert diluent carrier and optionally other active compounds.

12. A method of controlling fungi comprising applying thereto or a habitat thereof a fungicidally effective amount of a compound as claimed in any of claims 1 to 9 or when made by a method as claimed in claim 10, optionally as a composition as claimed in claim 11.

**Claims for the Contracting State: AT**

1. A fungicidal composition comprising a fungicidally effective amount of a compound of the general formula:—

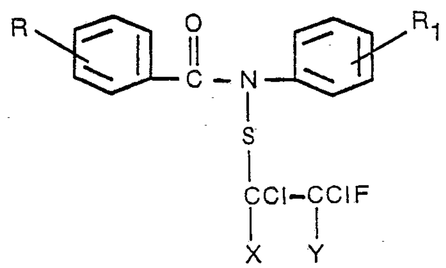

in which R is hydrogen, $C_1$—$C_5$ alkyl, $C_1$—$C_3$ haloalkyl, halogen, —$NO_2$, $C_1$—$C_2$ alkoxy, $R_1$ is —H or —C≡N, X and Y are either —Cl or —F provided they are not identical when $R_1$ is —H and X and Y are —Cl when $R_1$ is —C≡N in assocation with an inert diluent carrier and optionally other active compounds.
2. A composition as claimed in claim 1 characterised in that R is 2—I, $R_1$ is —H, X is —Cl and Y is —F.
3. A composition as claimed in claim 1 characterised in that R is —H, $R_1$ is —H, X is —Cl and Y is —F.
4. A composition as claimed in claim 1 characterised in that R is —H, $R_1$ is —H, X is —F and Y is —Cl.
5. A composition as claimed in claim 1 characterised in that R is 2—$CF_3$, $R_1$ is —H, X is —F and Y is —Cl.
6. A compositon as claimed in claim 1 characterised in that R is 2—$CF_3$, $R_1$ is —H, X is —Cl and Y is —F.
7. A compositon as claimed in claim 1 characterised in that R is 2—I, $R_1$ is —H, X is —F and Y is —Cl.
8. A composition as claimed in claim 1 characterised in that R is 2—$OCH_3$, $R_1$ is —H, X is —F and Y is —Cl.
9. A composition as claimed in claim 1 characterised in that R is 2—I, $R_1$ is —C≡N, X is —Cl, and Y is —Cl.
10. A method of making a compound as claimed in claim 1 wherein a compound of the general formula

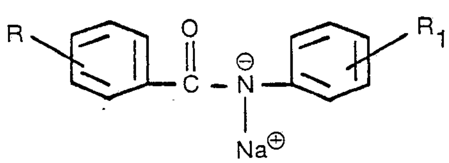

in which R and $R_1$ have the meaning given in claim 1, is reacted with a compound of the general formula

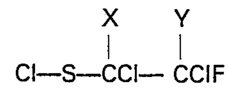

where X and Y have the meaning given in claim 1.

11. A method of controlling fungi comprising applying thereto or to a habitat thereof a fungicidally effective amount of a compound as defined in any of claims 1 to 9 or when made by a method as claimed in claim 10, optionally as a composition as claimed in any of claims 1—9.

**Revendications pour les Etats contractants: BE CH DE FR GB IT LI NL SE**

1. Un composé de formule générale:

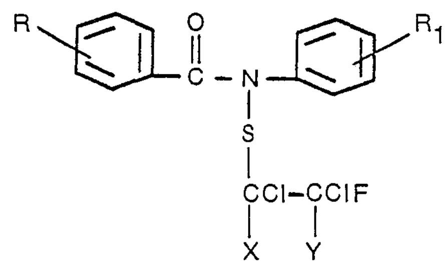

dans laquelle:

R représente un hydrogène, un alkyle en $C_1$ à $C_5$, un haloalkyle en $C_1$ à $C_3$, un halogène, $NO_2$, un alkoxy en $C_1$ à $C_2$;

$R_1$ représente —H ou —C≡N;

X et Y représentent soit —Cl soit —F, pourvu qu'ils ne soient pas identiques lorsque $R_1$ représente H et X et Y représent —Cl lorsque $R_1$ représente —C≡N.

2. Un composé selon la revendication 1, caractérisé en ce que R représente 2—I, $R_1$ représente H, X représente —Cl et Y représente —F.

3. Un composé selon la revendication 1, caractérisé en ce que R représente H, $R_1$ représente H, X représente —Cl et Y représente —F.

4. Un composé selon la revendication 1, caractérisé en ce que R représente H, $R_1$ représent H, X représente —F et Y représente —Cl.

5. Un composé selon la revendication 1, caractérisé en ce que R représente 2—$CH_3$, $R_1$ représente H, X représente —F et Y représente —Cl.

6. Un composé selon la revendication 1, caractérisé en ce que R représente 2—$CH_3$, $R_1$ représente H, X représente —Cl et Y représente —F.

7. Un composé selon la revendication 1, caractérisé en ce que R représente 2—I, $R_1$ représente H, X représente —F et Y représente —Cl.

8. Un composé selon la revendication 1, caractérisé en ce que R représente 2—$OCH_3$, $R_1$ représente H, X représente —F et Y représente —Cl.

9. Un composé selon la revendication 1, caractérisé en ce que R représente 2—I, $R_1$ représente —C≡N, X représente —Cl et Y représente —Cl.

10. Un procédé de préparation d'un composé selon la revendication 1, dans lequel on fait réagir un composé de formule générale:

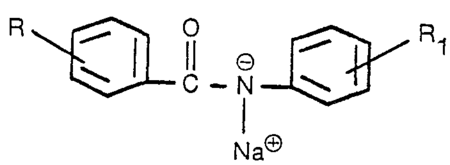

dans laquelle R et $R_1$ ont les significations indiquées dans la revendication 1, avec un composé de formule générale:

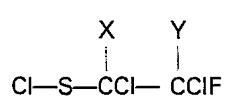

dans laquelle X et Y ont les significations indiquées dans la revendication 1.

11. Une composition fongicide comprenant une quantité efficace en tant que fongicide d'un composé selon l'une quelconque des revendications 1 à 9, ou un composé préparé selon la revendication 10 en association avec un véhicule diluant inerte et éventuellement d'autres composés actifs.

12. Un procédé de contrôle de champignons consistant à leur appliquer ou à appliquer à leur habitat une quantité efficace en tant que fongicide d'un composé selon l'une quelconque des revendications 1 à 9 ou d'un composé préparé par un procédé selon la revendication 10, éventuellement sous la forme d'une composition selon la revendication 11.

**Revendications pour l'Etat contractant: AT**

1. Une composition fongicide comprenant une quantité efficace en tant que fongicide d'un composé de formule générale:

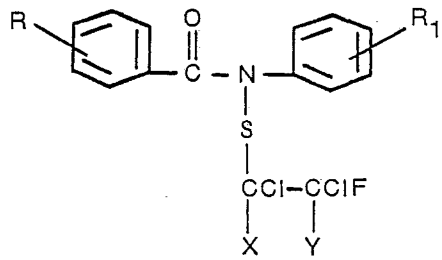

dans laquelle:

R représente un hydrogène, un alkyle en $C_1$ à $C_5$, un haloalkyle en $C_1$ à $C_3$, un halogène, $NO_2$, un alkoxy en $C_1$ à $C_2$;

$R_1$ représente —H ou —C≡N;

X et Y représentent soit —Cl soit —F, pourvu qu'ils ne soient pas identiques lorsque $R_1$ représente H et X et Y représent —Cl lorsque $R_1$ représente —C≡N, en association avec un véhicule diluant inerte et éventuellement d'autres composés actifs.

2. Un composition selon la revendication 1, caractérisée en ce que R représente 2—I, $R_1$ représente H, X représente —Cl et Y représente —F.

3. Un composition selon la revendication 1, caractérisée en ce que R représente H, $R_1$ représente H, X représente —Cl et Y représente —F.

4. Un composition selon la revendication 1, caractérisée en ce que R représente H, $R_1$ représent H, X représente —F et Y représente —Cl.

5. Un composition selon la revendication 1, caractérisée en ce que R représente 2—$CH_3$, $R_1$ représente H, X représente —F et Y représente —Cl.

6. Un composition selon la revendication 1, caractérisée en ce que R représente 2—$CH_3$, $R_1$ représente H, X représente —Cl et Y représente —F.

7. Un composition selon la revendication 1, caractérisée en ce que R représente 2—I, $R_1$ représente H, X représente —F et Y représente —Cl.

8. Une composition selon la revendication 1, caractérisée en ce que R représente 2—$OCH_3$, $R_1$ représente H, X représente —F et Y représente —Cl.

9. Un composition selon la revendication 1, caractérisée en ce que R représente 2—I, $R_1$ représente —C≡N, X représente —Cl et Y représente —Cl.

10. Un procédé de préparation d'un composé selon la revendication 1, caractérisé en ce que l'on fait réagir un composé de formule générale:

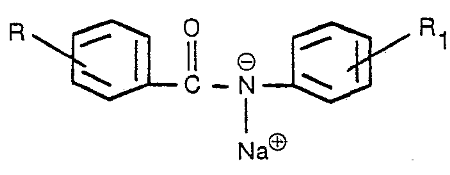

dans laquelle R et $R_1$ ont les mêmes significations que dans la revendication 1, avec un composé de formule générale:

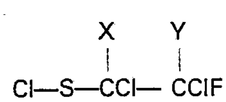

dans laquelle X et Y ont les significations indiquées dans la revendication 1.

11. Un procédé de contrôle de champignons consistant à leur appliquer ou à appliquer à leur habitat une quantité efficace en tant que fongicide d'un composé selon l'une quelconque des revendications 1 à 9 ou d'un composé préparé par un procédé selon la revendication 10, éventuellement sous la forme d'une composition selon l'une quelconque des revendications 1 à 9.

**Patentansprüche für die Vertragsstaaten: BE CH DE FR GB IT LI NL und SE**

1. Substanzen der allgemeinen chemischen Formel

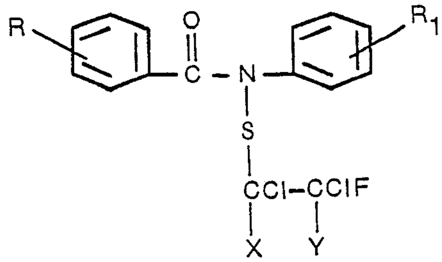

in der R Wasserstoff, $C_1$—$C_5$ Alyl, $C_1$—$C_3$ Halogenalkyl, Halogen, —$NO_2$, $C_1$—$C_2$ Alkoxy, $R_1$ Waserstoff oder —CN, X und Y entweder Chlor oder Fluor mit der Maßgabe sind, daß nicht beide Reste die gleiche Bedeutung haben, wenn $R_1$ Wasserstoff ist, und daß X und Y Chlor bedeuten, wenn $R_1$ —CN ist.

2. Substanz nach Anspruch 1, dadurch gekennzeichnet, daß R ein Iodatom in 2-Stellung, $R_1$ Wasserstoff, X Chlor und Y Fluor sind.

3. Substanz nach Anspruch 1, dadurch gekennzeichnet, daß R Wasserstoff, $R_1$ Wasserstoff, X Chlor und Y Fluor sind.

4. Substanz nach Anspruch 1, dadurch gekennzeichnet, daß R Wasserstoff, $R_1$ Wasserstoff, X Fluor und Y Chlor sind.

5. Substanz nach Anspruch 1, dadurch gekennzeichnet, daß R Trifluormethyl in 2-Stellung, $R_1$ Wasserstoff, X Fluor und Y Chlor ist.

6. Substanz nach Anspruch 1, dadurch gekennzeichnet, daß R Trifluormethyl in 2-Stellung, $R_1$ Wasserstoff, X Chlor und Y Fluor sind.

7. Substanz nach Anspruch 1, dadurch gekennzeichnet, daß R Iod in 2-Stellung, $R_1$ Wasserstoff, X Fluor und Y Chlor sind.

8. Substanz nach Anspruch 1, dadurch gekennzeichnet, daß R die Methoxygruppe in 2-Stellung, $R_1$ Wasserstoff, X Fluor un Y Chlor sind.

9. Substanz nach Anspruch 1, dadurch gekennzeichnet, daß R Iod in 2-Stellung, $R_1$ Cyano, X Chlor und Y Chlor sind.

10. Verfahren zur Herstellung einer Substanz nach Anspruch 1, dadurch gekennzeichnet, daß eine Substanz der allgemeinen chemischen Formel

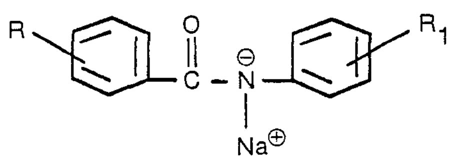

in der R und $R_1$ die im Anspruch 1 angegebene Bedeutung haben, mit einer Substanz der allgemeinen chemischen Formel

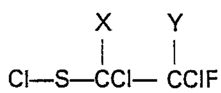

umgesetzt wird, in der X und Y die im Anspruch 1 angegebene Bedeutung haben.

11. Fungizid, enthaltend eine der Substanzen nach einem der Ansprüche 1 bis 9 in einer fungizid wirksamen Menge, oder soweit nach Anspruch 10 hergestellt, in Verbindung mit einem inerten verdünnenden Träger und gegebenenfalls wahlweise weiteren aktiven Substanzen.

12. Verfahren zur Unterdrückung des Pilzbefalls, dadurch gekennzeichnet, daß der Pilz oder sein Wucherbereich mit einer fungizid wirksamen Menge der Substanz nach einem der Ansprüche 1 bis 9, oder einer Substanz, die nach Anspruch 10 hergestellt worden ist, wahlweise als Gemisch nach Anspruch 11 formuliert, in Berührung gebracht werden.

**Patentansprüche für den Vertragsstaat: AT**

1. Fungizides Gemisch enthaltend eine fungizid wirksame Menge einer Substanz der allgemeinen chemischen Formel

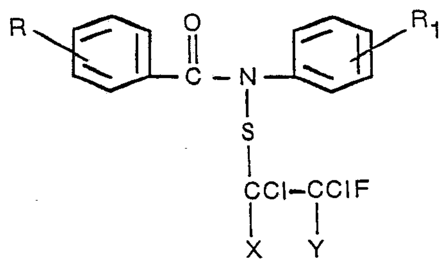

in der R Wasserstoff, $C_1$—$C_5$ Alkyl, $C_1$—$C_3$ Halogenalkyl, Halogen, —$NO_2$, $C_1$—$C_2$ Alkoxy, $R_1$ Wasserstoff oder —CN, X und Y entweder Chlor oder Fluor mit der Maßgabe sind, daß sie nicht beide die gleiche Bedeutung haben, wenn $R_1$ Wasserstoff ist, und X und Y Chlor sind, wenn $R_1$ die Cyanogruppe ist, in Verbindung mit einem inerten Verdünnungsträger und gegebenenfalls, anderen aktiven Substanzen.

2. Gemisch nach Anspruch 1, dadurch gekennzeichnet, daß R ein lod in 2-Stellung, $R_1$ Wasserstoff, X Chlor und Y Fluor sind.

3. Gemisch nach Anspruch 1, dadurch gekennzeichnet, daß R Wasserstoff, $R_1$ Wasserstoff, X Chlor und Y Fluor sind.

4. Gemisch nach Anspruch 1, dadurch gekennzeichnet, daß R Wasserstoff, $R_1$ Wasserstoff, X Fluor und Y Chlor sind.

5. Gemisch nach Anspruch 1, dadurch gekennzeichnet, daß R Trifluormethyl in 2-Stellung, $R_1$ Wasserstoff, X Fluor und Y Chlor sind.

6. Gemisch nach Anspruch 1, dadurch gekennzeichnet, daß R Trifluormethyl in 2-Stellung, $R_1$ Wasserstoff, X Chlor und Y Fluor sind.

7. Gemisch nach Anspruch 1, dadurch gekennzeichnet, daß R lod in 2-Stellung, $R_1$ Wasserstoff, X Fluor und Y Chlor sind.

8. Gemisch nach Anspruch 1, dadurch gekennzeichnet, daß R die Methoxygruppe in 2-Stellung, $R_1$ Wasserstoff, X Fluor un Y Chlor sind.

9. Gemisch nach Anspruch 1, dadurch gekennzeichnet, daß R lod in 2-Stellung, $R_1$ Cyano, X Chlor und Y Chlor sind.

10. Verfahren zur Herstellung einer Substanz nach Anspruch 1, dadurch gekennzeichnet, daß eine Verbindung der allgemeinen chemischen Formel

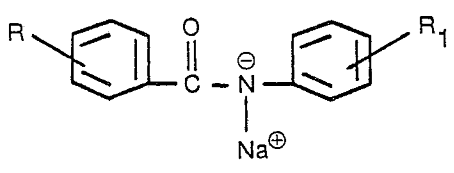

in der R und $R_1$ die im Anspruch 1 angegebene Bedeutung haben, mit einer Substanz der allgemeinen chemischen Formel

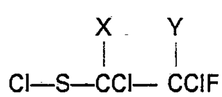

umgesetzt wird, in der X und Y die im Anspruch 1 genannte Bedeutung haben.

11. Verfahren zur Unterdrückung des Pilzbefalls, dadurch gekennzeichnet, daß auf den Pilz oder seinen Ausbreitungsbereich eine fungizid wirksame Menge einer Substanz, wie sie in einem der Ansprüche 1 bis 9 definiert ist, oder wie sie nach Anspruch 10 hergestellt worden ist, wahlweise als Gemisch nach einem der Ansprüche 1 bis 9 zur Einwirkung gegebracht wird.